# EUROPEAN PATENT SPECIFICATION

(11) **EP 3 938 168 B1**
(45) Date of publication and mention of the grant of the patent: **31.08.2022**
(21) Application number: 20709570.4
(22) Date of filing: 06.03.2020
(51) Int. Cl.: B29C 48/285, H05H 1/24, A61L 2/14, A61L 11/00, C08J 3/28, B29B 7/90, B29B 7/46, B29B 7/92, B29B 9/06, B29B 9/14, B29C 48/40

(54) **PROCESS FOR MAKING COMPOUNDS USING WASTES OF NATURAL ORIGIN AND FIBRES OF PLANT OR ANIMAL ORIGIN**
VERFAHREN ZUR HERSTELLUNG VON VERBINDUNGEN UNTER VERWENDUNG VON ABFÄLLEN NATÜRLICHEN URSPRUNGS UND FASERN PFLANZLICHEN ODER TIERISCHEN URSPRUNGS
PROCÉDÉ DE FABRICATION DE COMPOSÉS UTILISANT DES DÉCHETS D'ORIGINE NATURELLE ET DES FIBRES D'ORIGINE VÉGÉTALE OU ANIMALE

(30) Priority: 12.03.2019 IT 201900003565
(43) Date of publication of application: 19.01.2022
(73) Proprietor: Mixcycling Srl, 36042 Vicenza, Breganze (VI) (IT)
(72) Inventor: TAGLIAPIETRA, Gianni, 36042 Vicenza (IT)
(74) Representative: Bettello, Pietro
(86) International application number: PCT/EP2020/056024
(87) International publication number: WO 2020/182660

(56) References cited:
- EP-A1- 2 565 004
- DE-A1-102013 101 667
- JP-A- 2001 239 239
- US-A1- 2006 257 299
- TATAROVA E ET AL: "Plasmas for environmental issues: from hydrogen production to 2D materials assembly", PLASMA SOURCES SCIENCE AND TECHNOLOGY, INSTITUTE OF PHYSICS PUBLISHING, BRISTOL, GB, vol. 23, no. 6, 13 October 2014 (2014-10-13), page 63002, XP020274318, ISSN: 0963-0252, DOI: 10.1088/0963-0252/23/6/063002 [retrieved on 2014-10-13]

## Description

The present invention relates to a process for making compounds using wastes of natural origin and fibres of plant or animal origin, according to the general part of claim 1.

As is well known in the industrial field, the management of processing wastes of natural origin, in particular those consisting of fibres of animal origin such as, for example, hides and those consisting of fibres of plant origin such as, for example, cork, wood, straw, cereal bran, spent coffee, hemp, cellulose, bamboo fibres, shells of walnuts and hazelnuts, pomace, rice husks for which it is difficult to find a suitable reuse as biomass, so they become waste products, with the consequent economic and ecological difficulties that arise for their disposal.

In the current state of the art, one method for recovering wastes is the one that envisages agglomerating the mass with adhesives, or with plastic materials, to obtain materials and semi-finished products that are used in various branches of industry. Semi-finished products consisting of slabs obtained with wood-cellulosic residues are mentioned for exemplary purposes which, in the construction sector, are used as thermal insulators, while in the furniture sector they replace the portions normally made of solid wood.

In practice, the products currently on the market, known with the generic term "compound", obtained by agglomerating the material deriving from wastes or natural fibres with adhesives of various types, show drawbacks that limit their use. Specifically, a first drawback is constituted by the fact that, in order to avoid the disintegration of the material, it is necessary to use a little "charge", that is to use a limited quantity of waste product or fibre, which entails the impossibility of making medium- and large-sized products, that is blocks or bales.

A second drawback derives from the fact that the semi-finished products obtained by agglomeration of wastes and natural fibres are considered to be commercially of low value, therefore low-quality adhesives are normally used to contain the cost and are not subjected to optimal sanitization process.

The well-known "pellets" are mentioned, used as domestic fuel for exemplary purposes which, for economic reasons, are made up of various wastes of plant fibres, from wood to cereals, which are agglomerated with poor quality adhesive, so when they burn emit harmful and polluting fumes.

In the documents EP 2565004 A1 and DE 102013101667 A1, which constitute the most relevant documents of the state of the art, processes are described for making compounds using wastes of natural origin and fibres of plant or animal origin, all according to the general part of the claim 1.

The documents US 2006/257299 A1 and JP 2001239239 A1 describe processes that use the technological process called "Non-thermal plasma" or "NTP", for the treatment of gaseous emissions to eliminate bad odours from industrial contaminants and organic wastes, respectively.

The scientific disclosure of the Bristol Institute of Physics under the title "Plasmas for environmental issues: from hydrogen production to 2D materials assembly" in the of Teterova et al. describes a process that uses "Non-thermal plasma" to remove contaminants from wastewater.

The scientific disclosure having the title "Innovative non-thermal plasma disinfection process inside sealed bags: assessment of bactericidal and sporicidal effectiveness in regard to current sterilization norms" in the name of Zouhaier Ben Belgacem et al. is also mentioned.

The purpose of the present invention is to provide a process for processing wastes of natural origin and fibres of plant or animal origin, of the type which provides for the agglomeration of the components which is free from the drawbacks manifested by similar processes of known type.

In particular, a purpose of the invention is to provide a process for making agglomerates based on natural, animal and plant wastes and fibres, known with the term "compound", with which it is possible to obtain semi-finished products that have physical characteristics and better mechanical properties than similar products of known type.

This purpose is achieved with the implementation of a process according to the characterizing part of claim 1.

In the dependent claims, particular embodiments of this process are provided.

The whole is better defined by the description of the processing steps of the process according to the invention.

The complete process of the invention substantially comprises the following steps in succession:
- step I: preparation of the "charge";
- step II: selection of the carriers and agglomerating additives;
- step III: preparation of the blend;
- step IV: processing the blend to obtain the compound;
- step V: processing the compound to obtain a semi-finished product.

Specifically, each step provides for the following operations:

### step I: preparation of the "charge"

- 1.1 Recovery of the wastes of natural origin and plant or animal fibres which results in the "charge" to be processed;
- 1.2 Drying of the waste material recovered to give the mass a consistency suitable for the subsequent processing;
- 1.3 Milling/grinding of the waste material to take the shredded mass to dimensions suitable for the subsequent processing;
- 1.4 Dimensional sieving to divide the shredded material into various sizes based on the mechanical properties and on the different aesthetic effects that the final compound must have (by way of example, the series of sizes can have dimensions <0.2 mm .; 0.2 -0.5 mm .; 0.5-1.0 mm .; 1.0-2.0 mm .; 2.0-4.0 mm);
- 1.5 Gravimetric sieving to separate, within a same dimensional size, elements with different weight, the lighter ones from the heavier ones, based on the mechanical properties and on the different aesthetical effects that the final compound must have; (by way of example the waste product known as "cork flour" can contain cork particles or bark particles which, although having the same dimensions as the particles of cork flour, have considerably different characteristics);
- 1.6 Sanitization, a fundamental step due to the fact that a material subject to bacterial contamination is processed, which is based on the principle of advanced oxidation which is carried out through the innovative technology called "Non-thermal plasma" or "NTP", which uses the so-called " non-thermal discharges with dielectric-barrier method "or" DBD ".

Operationally, the ionization potential and the density of the charge species generated by the plasma with electric barrier charge or "DBD" are greater than those present in the non-thermal plasma generated with other systems, for which a beneficial treatment is achieved which strongly reduces, until elimination, the bacterial charges, the volatile organic substances (VOC) are decomposed and the odours are eliminated, and in addition, in all this, no chemical product is used, thus giving rise to a totally ecological treatment.

### Step II: selection of the carriers and agglomerating additives

- 2.1 Carriers, plastic materials used to agglomerate natural fibres such as to obtain a well cohesive compound, composed of non-biodegradable plastics, both of fossil origin and bio-based (Polyethylene, Polypropylene, Polystyrene, PMMA, PET, SBS rubbers and SEBS, abs, methacrylate are mentioned for exemplary purposes);
   or biodegradable plastics, both of fossil origin and bio-based (PLA (polylactic acid) is mentioned for exemplary purposes), plastics derived from corn starch, potatoes, vegetable oils;
- 2.2 Additives with agglomerating function, composed of modified polymeric adhesives having the function of grafting and considerably increasing the adhesion between the plant or animal material (charge) and polymers (carriers), such as to allow to introduce a higher amount of fibres into the blend and with antibacterial function, based on silver ions.
   Operationally, a PLA (polylactic acid) modified with MAH (maleic anhydride) is used with biodegradable materials, while with non-biodegradable materials, polymers modified based on PP (polypropylene), SEBS (styrene-ethylene-butylene-styrene), PE (polyethylene in all forms thereof), all grafted with MAH (maleic anhydride) are used.

### Step III: preparation of the blend

- mixture consisting of the charge, carriers and agglomerating additives in which the percentages of the three components vary based on the type of grafting wanted and the mechanical/physical characteristics that the compound or the semi-finished final product must have; operationally, the blend is a mixture that can comprise: charges from 10% to 80% - carriers from 20% to 90% - additives from 1% to 5%, in the various combinations.

### step IV: processing the blend to obtain the compound

- 4.1 Preparation of the raw materials (charges, carriers, agglomerating additives) by means of molecular-sieve dehumidifiers, able to ensure a correct humidity down into the inside of the particles (granules or fibres), in order to increase adhesion between the components and prevent the creation of condensation during the compound processing steps;
- 4.2 Mixing: the various components which are "dry blend" mixed with specific mixers or, in alternative, using volumetric or gravimetric dosing devices which mix the various components directly inside the machine.

### step V: processing the compound to obtain a semi-finished product

- 5.1 Extrusion: a specific twin-screw extruder which mixes homogeneously the components, without stressing or burning fibres, and, by means of a drawing head, makes filaments/strands with a diameter comprised between 3 mm and 5 mm which are, through a cutter, shredded into granules (pellets) and, thereafter, are sieved to be divided based on the weight or on predefined sizes;
- 5.2 Sanitization of the granules: it is carried out through the innovative technology called "Non-thermal Plasma" or "NTP", which uses the so-called "non-thermal discharges with dielectric-barrier method" or "DBD", equal to the one used and described for sanitizing the "charge";
- 5.3 Packaging: pellets are packaged in protection bags under "NTP"-modified atmosphere (Non Thermal-Plasma), to make the product more salubrious and increase its "shelf life".

## Claims

1. PROCESS FOR MAKING COMPOUNDS USING WASTES OF NATURAL ORIGIN AND FIBRES OF PLANT OR ANIMAL ORIGIN, wherein wastes of natural origin, fibres of plant origin, as well as wastes of animal origin, composing the so-called "charge", are mixed with agglomerating plastic materials, the so-called "carriers", and with agglomerating additives in order to form a mixture, the so-called "blend", which is transformed into a compound, the "compound", used for making semi-finished products, said process providing the following sequenced steps:
- step I: preparation of the "charge";
- step II: selection of the "carriers" and agglomerating additives;
- step III: preparation of the "blend";
- step IV: processing the "blend" to obtain the "compound";
- step V: processing the "compound" to obtain a semi-finished product
**said process being characterized in that**
the initial "charge" is submitted to a sanitization treatment, based on the principle of advanced oxidation, obtained by applying the technological process referred to as "Non-thermal plasma" or "NTP", where the so-called "non-thermal discharges with dielectric-barrier method" or "DBDs" are used, in order to strongly reduce bacterial charges, until removal thereof, decompose the volatile organic substances (VOCs) and remove smells.

2. PROCESS, according to claim 1, **characterized in that** the final semi-finished product obtained with the "compound" is submitted to a sanitization treatment, based on the principle of advanced oxidation, obtained by applying the technological process referred to as "NTP", where the so-called "DBDs" are used.

3. PROCESS, according to claim 1, **characterized in that** the step I comprises the following processing:
- recovery of the wastes of natural origin and plant or animal fibres, from the factories producing wastes and which results in the "charge";
- drying of the waste material recovered to give the mass a consistency suitable for the subsequent processing;
- milling/grinding of the waste material to take the shredded mass to dimensions suitable for the subsequent processing.
- dimensional sieving to divide the shredded material into various sizes based on the mechanical properties and on the different aesthetic effects that the final "compound" must have;
- gravimetric sieving to separate, within a same dimensional size, elements with different weight, the lighter ones from the heavier ones, based on the mechanical properties and on the different aesthetical effects that the final "compound" must have;
- sanitization, to strongly reduce the bacterial charges, until removal thereof, decompose the volatile organic substances (VOCs) and remove smells.

4. PROCESS, according to claim 1, **characterized in that** the step II comprises the following processing:
- selection of the "carriers", plastic materials used to agglomerate natural fibres such as to obtain a well cohesive "compound", composed of non-biodegradable plastics, both of fossil origin and bio-based (Polyethylene, Polypropylene, Polystyrene, PMMA, PET, SBS rubbers and SEBS, abs, methacrylate are mentioned for exemplary purposes), or biodegradable plastics, both of fossil origin and bio-based (PLA (polylactic acid) is mentioned for exemplary purposes), plastics derived from corn starch, potatoes, vegetable oils;
- selection of the additives: those with agglomerating function, composed of modified polymeric adhesives having the function of grafting and considerably increasing the adhesion between the plant or animal material (charge) and polymers (carriers), such as to allow to introduce a higher amount of fibres into the "blend" and where a PLA (polylactic acid) modified with MAH (maleic anhydride) is used with biodegradable materials, while with non-biodegradable materials, polymers modified based on PP (polypropylene), SEBS (styrene-ethylene-butylene-styrene), PE (polyethylene in all forms thereof), all grafted with MAH (maleic anhydride) and those with antibacterial function, based on silver ions, are used.

5. PROCESS, according to claim 1, **characterized in that** the step III consists in preparing the "blend", a mixture composed of the "charge", "carriers" and agglomerating additives, where the percentages of the three components vary based on the intended type of graft and on the mechanical/physical features that the "compound" or the final semi-finished product must have;

6. PROCESS, according to claim 1, **characterized in that** the step IV, consisting in processing the "blend" to obtain the "compound", includes the following steps:
- preparation of the raw materials (charges, carriers, agglomerating additives) by means of molecular-sieve dehumidifiers, able to ensure a correct humidity down into the inside of the particles (granules or fibres), in order to increase adhesion between the components and prevent the creation of condensation during the "compound" processing steps;
- mixing of the various components which are "dry blend" mixed with specific mixers or, in alternative, using volumetric or gravimetric dosing devices which mix the various components directly inside the machine.

7. PROCESS, according to claim 1, **characterized in that** the step V, which consists in processing the "compound" to obtain a semi-finished product, includes:
- extrusion by means of a twin-screw extruder which mixes homogeneously the components, without stressing or burning fibres, and, by means of a drawing head, makes filaments/strands which are shredded into granules (pellets) and, thereafter, are sieved to be divided based on the weight or on predefined sizes;
- sanitization of the granules which is based on the principle of advanced oxidation, obtained by applying the technological process referred to as "NTP", where the so-called "DBDs" are used.
- packaging of pellets in protection bags under "NTP"-modified atmosphere, to make the product more salubrious and increase its "shelf life".

## Patentansprüche

1. VERFAHREN ZUR HERSTELLUNG VON VERBINDUNGEN UNTER VERWENDUNG VON ABFÄLLEN NATÜRLICHEN URSPRUNGS UND FASERN PFLANZLICHEN ODER TIERISCHEN URSPRUNGS, wobei Abfälle natürlichen Ursprungs, Fasern pflanzlichen Ursprungs sowie Abfälle tierischen Ursprungs, die die sogenannte "Charge" bilden, mit agglomerierenden Kunststoffmaterialien, den sogenannten "Trägerstoffen", und mit agglomerierenden Zusatzstoffen gemischt werden, um ein Gemisch, die sogenannte "Mischung", zu bilden, das in eine Verbindung, die "Verbindung", umgewandelt wird, die zur Herstellung von Halbfertigprodukten verwendet wird, wobei das Verfahren die folgenden aufeinanderfolgenden Schritte vorsieht:
- Schritt I: Vorbereitung der "Charge";
- Schritt II: Auswahl der "Trägerstoffe" und der agglomerierenden Zusatzstoffe;
- Schritt III: Vorbereitung der "Mischung";
- Schritt IV: Verarbeitung der "Mischung", um die "Verbindung" zu erhalten;
- Schritt V: Verarbeitung der "Verbindung", um ein Halbfertigprodukt zu erhalten
**wobei das Verfahren dadurch gekennzeichnet ist, dass**
die anfängliche "Charge" einer Desinfektionsbehandlung unterzogen wird, die auf dem Prinzip der fortgeschrittenen Oxidation beruht und durch die Anwendung des technologischen Verfahrens erhalten wird, das als "Nichtthermisches Plasma" oder "NTP" bezeichnet wird, bei dem die sogenannten "nichtthermischen Entladungen mit dielektrischer Barriere-Methode" oder "DBD" verwendet werden, um die bakteriellen Ladungen bis zu deren Entfernung stark zu verringern, die flüchtigen organischen Stoffe (VOC) zu zersetzen und Gerüche zu entfernen.

2. VERFAHREN nach Anspruch 1, **dadurch gekennzeichnet, dass** das mit der "Verbindung" erhaltene endgültige Halbfertigprodukt einer Desinfektionsbehandlung unterzogen wird, die auf dem Prinzip der fortgeschrittenen Oxidation beruht und durch Anwendung des als "NTP" bezeichneten technologischen Verfahrens erhalten wird, bei dem die sogenannten "DBDs" verwendet werden.

3. VERFAHREN nach Anspruch 1, **dadurch gekennzeichnet, dass** der Schritt I die folgende Verarbeitung umfasst:
- Verwertung der Abfälle natürlichen Ursprungs und pflanzlicher oder tierischer Fasern aus den Betrieben, die Abfälle erzeugen, was eine "Charge" ergibt;
- Trocknung des verwerteten Abfallmaterials, um der Masse eine für die nachfolgende Verarbeitung geeignete Konsistenz zu verleihen;
- Fräsen/Schleifen des Abfallmaterials, um die zerkleinerte Masse auf eine für die nachfolgende Verarbeitung geeignete Größe zu bringen.
- dimensionales Sieben, um das zerkleinerte Material in verschiedene Größen zu unterteilen, die auf den mechanischen Eigenschaften und den verschiedenen ästhetischen Effekten beruhen, die die endgültige "Verbindung" aufweisen muss;
- gravimetrisches Sieben, um innerhalb einer gleichen Maßgröße Elemente mit unterschiedlichem Gewicht zu trennen, die leichteren von den schwereren, basierend auf den mechanischen Eigenschaften und den unterschiedlichen ästhetischen Effekten, die die endgültige "Verbindung" aufweisen muss;
- Desinfektion, um die bakteriellen Ladungen bis zu deren Entfernung stark zu reduzieren, die flüchtigen organischen Substanzen (VOCs) zu zersetzen und Gerüche zu entfernen.

4. VERFAHREN nach Anspruch 1, **dadurch gekennzeichnet, dass** der Schritt II die folgende Verarbeitung umfasst:
- Auswahl der "Trägerstoffe", Kunststoffmaterialien, die zur Agglomeration von Naturfasern verwendet werden, um eine gut kohäsive "Verbindung" zu erhalten, bestehend aus biologisch nicht abbaubaren Kunststoffen, sowohl fossilen Ursprungs als auch auf biologischer Basis (Polyethylen, Polypropylen, Polystyrol, PMMA, PET, SBS-Kautschuke und SEES, Abs, Methacrylat werden beispielhaft genannt), oder biologisch abbaubaren Kunststoffen, sowohl fossilen Ursprungs als auch auf biologischer Basis (PLA (Polymilchsäure) wird beispielhaft genannt), Kunststoffe aus Maisstärke, Kartoffeln, Pflanzenölen;
- Auswahl der Zusatzstoffe: solche mit agglomerierender Funktion, die aus modifizierten polymeren Klebstoffen bestehen, die die Funktion der Pfropfung absolvieren und die Adhäsion zwischen dem pflanzlichen oder tierischen Material (Charge) und den Polymeren (Trägerstoffen) beträchtlich erhöhen, so dass eine höhere Menge an Fasern in die "Mischung" eingebracht werden kann, und wobei bei biologisch abbaubaren Materialien ein mit MAH (Maleinsäureanhydrid) modifiziertes PLA (Polymilchsäure) verwendet wird, während bei biologisch nicht abbaubaren Materialien modifizierte Polymere auf der Basis von PP (Polypropylen), SEES (Styrol-Ethylen-Butylen-Styrol), PE (Polyethylen in allen seinen Formen), die alle mit MAH (Maleinsäureanhydrid) gepfropft sind, und solche mit antibakterieller Funktion auf der Basis von Silberionen verwendet werden.

5. VERFAHREN nach Anspruch 1, **dadurch gekennzeichnet, dass** der Schritt III darin besteht, die "Mischung", ein Gemisch, das aus der "Charge", den "Trägerstoffen" und den agglomerierenden Zusatzstoffen besteht, vorzubereiten, wobei die Prozentsätze der drei Komponenten je nach der beabsichtigten Art der Pfropfung und den mechanischen/physikalischen Eigenschaften, die die "Verbindung" oder das endgültige Halbfertigprodukt aufweisen muss, variieren;

6. VERFAHREN nach Anspruch 1, **dadurch gekennzeichnet, dass** der Schritt IV, der darin besteht, die "Mischung" zu verarbeiten, um die "Verbindung" zu erhalten, die folgenden Schritte beinhaltet:
- Vorbereitung der Rohstoffe (Chargen, Trägerstoffe, agglomerierende Zusatzstoffe) mit Hilfe von Molekularsieb-Entfeuchtern, die in der Lage sind, eine korrekte Feuchtigkeit bis ins Innere der Partikel (Granulate oder Fasern) zu gewährleisten, um die Adhäsion zwischen den Komponenten zu erhöhen und die Bildung von Kondenswasser während der Verarbeitungsschritte der "Verbindung" zu verhindern;
- Mischung der verschiedenen Komponenten, die mit speziellen Mischern "trocken gemischt" werden, oder alternativ unter Verwundung von volumetrischen oder gravimetrischen Dosiergeräten, die die verschiedenen Komponenten direkt in der Maschine mischen.

7. VERFAHREN nach Anspruch 1, **dadurch gekennzeichnet, dass** der Schritt V, der darin besteht, die "Verbindung" zu verarbeiten, um ein Halbfertigprodukt zu erhalten, beinhaltet:
- Extrusion mittels eines Doppelschneckenextruders, der die Komponenten homogen mischt, ohne die Fasern zu belasten oder zu verbrennen, und mittels eines Ziehkopfes,
stellt Fäden/Stränge her, die zu Granulaten (Pellets) zerkleinert und anschließend gesiebt werden, um sie nach Gewicht oder vordefinierten Größen zu trennen;
- Desinfektion der Granulate, die auf dem Prinzip der fortgeschrittenen Oxidation beruht, die durch Anwendung des als "NTP" bezeichneten technologischen Verfahrens erhalten wird, wenn die sogenannten "DBDs" verwendet werden.
- Verpackung der Pellets in Schutzsäcken unter "NTP"-modifizierter Atmosphäre, um das Produkt bekömmlicher herzustellen und seine "Haltbarkeit" zu erhöhen.

## Revendications

1. PROCÉDÉ DE FABRICATION DE COMPOSÉS UTILISANT DES DÉCHETS D'ORIGINE NATURELLE ET DES FIBRES D'ORIGINE VÉGÉTALE OU ANIMALE, dans lequel les déchets d'origine naturelle, les fibres d'origine végétale, ainsi que les déchets d'origine animale, composant ladite « charge », sont mélangés avec des matières plastiques agglomérantes, lesdits « supports », et avec des additifs agglomérants afin de former un mélange, ledit « mélange », qui est transformé en un composé, le « composé », utilisé pour fabriquer des produits semi-finis, ledit procédé comprenant les étapes séquentielles suivantes :
- étape I : préparation de la « charge » ;
- étape II : sélection des « supports » et des additifs agglomérants ;
- étape III : préparation du « mélange » ;
- étape IV : traitement du « mélange » pour obtenir le « composé » ;
- étape V : traitement du « composé » pour obtenir un produit semi-fini
**ledit procédé étant caractérisé en ce que**
la « charge » initiale est soumise à un traitement d'assainissement, basé sur le principe de l'oxydation avancée, obtenu en appliquant le processus technologique appelé « plasma non thermique » ou « NTP », où sont utilisées lesdites « décharges non thermiques avec méthode à barrière diélectrique » ou « DBD », afin de réduire fortement les charges bactériennes, jusqu'à leur élimination, de décomposer les substances organiques volatiles (COV) et d'éliminer les odeurs.

2. PROCÉDÉ, selon la revendication 1, **caractérisé en ce que** le produit semi-fini final obtenu avec le « composé » est soumis à un traitement d'assainissement, basé sur le principe de l'oxydation avancée, obtenu en appliquant le processus technologique appelé « NTP », où lesdites « DBD » sont utilisées.

3. PROCÉDÉ, selon la revendication 1, **caractérisé en ce que** l'étape I comprend le traitement suivant :
- la récupération des déchets d'origine naturelle et des fibres végétales ou animales, auprès des usines qui produisent des déchets et qui permettent d'obtenir la « charge » ;
- le séchage des déchets récupérés afin de donner à la masse une consistance adaptée au traitement ultérieur ;
- le fraisage/broyage des déchets pour amener la masse déchiquetée à des dimensions adaptées au traitement ultérieur.
- le tamisage dimensionnel pour diviser le matériau déchiqueté en différentes tailles en fonction des propriétés mécaniques et des différents effets esthétiques que doit avoir le « composé » final ;
- le tamisage gravimétrique pour séparer, à l'intérieur d'une même dimension, des éléments de poids différents, les plus légers des plus lourds, en fonction des propriétés mécaniques et des différents effets esthétiques que doit avoir le « composé » final ;
- l'assainissement, pour réduire fortement les charges bactériennes, jusqu'à leur élimination, décomposer les substances organiques volatiles (COV) et éliminer les odeurs.

4. PROCÉDÉ, selon la revendication 1, **caractérisé en ce que** l'étape II comprend le traitement suivant :
- la sélection des « supports », matières plastiques utilisées pour agglomérer les fibres naturelles de manière à obtenir un « composé » bien cohésif, composé de plastiques non biodégradables, tant d'origine fossile que d'origine biologique (le polyéthylène, le polypropylène, le polystyrène, le PMMA, le PET, les caoutchoucs SBS et le SEES, l'abs, le méthacrylate sont mentionnés à titre d'exemple), ou de plastiques biodégradables, tant d'origine fossile que d'origine biologique (le PLA (acide polylactique) est mentionné à titre d'exemple), de plastiques dérivés de l'amidon de maïs, de pommes de terre, d'huiles végétales ;
- la sélection des additifs : ceux à fonction agglomérante, composés d'adhésifs polymères modifiés ayant pour fonction de greffer et d'augmenter considérablement l'adhésion entre la matière végétale ou animale (charge) et les polymères (supports), de manière à permettre d'introduire une plus grande quantité de fibres dans le « mélange » et où l'on utilise un PLA (acide polylactique) modifié avec du MAH (anhydride maléique) avec des matériaux biodégradables, tandis qu'avec les matériaux non biodégradables, on utilise des polymères modifiés à base de PP (polypropylène), de SEES (styrène-éthylène-butylène-styrène), de PE (polyéthylène sous toutes ses formes), tous greffés avec du MAH (anhydride maléique) et ceux ayant une fonction antibactérienne, à base d'ions argent.

5. PROCÉDÉ, selon la revendication 1, **caractérisé en ce que** l'étape III consiste à préparer le « mélange », un mélange composé de la « charge », des « supports » et des additifs agglomérants, où les pourcentages des trois composants varient en fonction du type de greffon prévu et des caractéristiques mécaniques/physiques que doit présenter le « composé » ou le produit semi-fini final ;

6. PROCÉDÉ, selon la revendication 1, **caractérisé en ce que** l'étape IV, consistant à traiter le « mélange » pour obtenir le « composé », comprend les étapes suivantes :
- la préparation des matières premières (charges, supports, additifs agglomérants) au moyen de déshumidificateurs à tamis moléculaire, capables d'assurer une humidité correcte jusqu'à l'intérieur des particules (granulés ou fibres), afin d'augmenter l'adhérence entre les composants et d'empêcher la création de condensation pendant les étapes de traitement de « composé » ;
- le mélange des différents composants qui sont mélangés « à sec » avec des mélangeurs spécifiques ou, en alternative, en utilisant des dispositifs de dosage volumétrique ou gravimétrique qui mélangent les différents composants directement à l'intérieur de la machine.

7. PROCÉDÉ, selon la revendication 1, **caractérisé en ce que** l'étape V, qui consiste à traiter le « composé » pour obtenir un produit semi-fini, comprend :
- l'extrusion au moyen d'une extrudeuse à double vis qui mélange de manière homogène les composants, sans stresser ni brûler les fibres, et, au moyen d'une tête d'étirage, fabrique des filaments/brins qui sont déchiquetés en granulés (pellets) et, ensuite, sont tamisés pour être divisés en fonction du poids ou de tailles prédéfinies ;
- l'assainissement des granulés qui est basée sur le principe de l'oxydation avancée, obtenue en appliquant le processus technologique appelé « NTP », où sont utilisées lesdites « DBD ».
- le conditionnement des granulés dans des sacs de protection sous atmosphère modifiée « NTP », pour rendre le produit plus salubre et augmenter sa « durée de vie ».
